# DEMANDE DE BREVET EUROPEEN

(11) **EP 0 999 197 A2**
(43) Date de publication de la demande: **10.05.2000**
(21) Numéro de dépôt: 99402486.7
(22) Date de dépôt: 11.10.1999
(51) Int. Cl.: C07C 29/149, C07H 15/04

(54) **Procédé de conversion de sucres oxydés en sucres hydrogénés par hydrogénation catalytique**

(30) Priorité: 13.10.1998 FR 9812791
(71) Demandeur: Roquette Frères, 62136 Lestrem (FR)
(72) Inventeur: Fleche, Guy, 59190 Hazebrouk (FR); Fuertes, Patrick, 59130 Lambersart (FR); Tamion, Rodolphe, 62157 Allouagne (FR)
(74) Mandataire: Boulinguiez, Didier

(57) **Abrégé**

L'invention a pour objet un nouveau procédé de conversion de sucres oxydés en sucres hydrogénés selon lequel le traitement d'hydrogénation est effectué en présence d'un promoteur de sélectivité constitué par une base de Lewis. Celle-ci peut avantageusement être choisie dans le groupe des quinones et notamment parmi l'acide anthraquinone-2 sulfonique et ses sels, en particulier son sel de sodium ou "AMS".

L'invention concerne également l'utilisation, en vue de l'hydrogénation catalytique de sucres oxydés, de catalyseurs à base notamment de ruthénium, préparés par une technique d'échange ionique et/ou par une technique d'apport d'un promoteur de sélectivité constitué par une base de Lewis telle que l'AMS.

## Description

La présente invention a pour objet un nouveau procédé de conversion de sucres oxydés en sucres hydrogénés, par hydrogénation catalytique. Elle vise tout particulièrement l'utilisation dudit procédé pour la préparation de sucres hydrogénés tels que érythritol, thréitol, ribitol, xylitol, arabitol, mannitol, sorbitol, iditol, maltitol ou lactitol. La présente invention concerne également l'utilisation, aux mêmes fins, d'un catalyseur d'hydrogénation particulier préparé dans des conditions spécifiques.

L'intérêt industriel et commercial, notamment dans les domaines alimentaire et pharmaceutique, des sucres hydrogénés tels que ceux précités, a été largement décrit dans la littérature, en particulier depuis deux décennies. On peut, à ce titre, se référer aux brevets publiés récemment au nom de la Demanderesse sous les numéros EP 490.768, EP 512.910, EP 518.770, EP 625.311, EP 630.573, EP 645.096, EP 711.743, EP 735.042, EP 774.210, EP 810.292, EP 832.899 et WO 95/02967.

Les besoins en sucres hydrogénés, en particulier en mono- et disaccharides hydrogénés, sont aujourd'hui tels que l'on recherche de nouveaux moyens industriels de préparation de ces composés, notamment à partir de co-produits agricoles ou industriels qui sont peu ou mal valorisés. Parmi les voies possibles de valorisation de tels co-produits, une attention toute particulière a été portée récemment aux procédés de conversion de sucres oxydés (ou de mélanges de sucres et de sucres oxydés) en sucres hydrogénés, impliquant au moins une étape d'hydrogénation catalytique.

Ainsi, la demande de brevet WO 93/19030 décrit un procédé de production de xylitol, seul ou en mélange avec de l'arabitol et du ribitol, par hydrogénation catalytique d'une composition intermédiaire contenant, en tant que sucre(s) oxydé(s), de l'acide xylonique, de l'acide arabinonique et/ou de l'acide lyxonique, lesdits acides se présentant sous la forme de lactones.

Selon les exemples de ce brevet, l'hydrogénation est avantageusement effectuée à une température de 130°C, à une pression d'hydrogène de 80 bars (8 MPa) et en présence d'un catalyseur à base soit de ruthénium sur charbon actif, soit de nickel de Raney. Cependant, quel que soit le catalyseur utilisé, la sélectivité de la réaction est limitée, à savoir au plus égale à 90-91%. Ceci, y compris lorsque le sucre oxydé, par exemple l'acide xylonique, a été préalablement purifié par plusieurs traitements successifs visant à le transformer en l'une des lactones correspondantes, en l'occurrence la L-xylono-1,4-lactone. Celle-ci est ensuite cristallisée en vue d'en éliminer les traces résiduelles d'acide formique considéré comme un "poison" du catalyseur d'hydrogénation au ruthénium.

Le brevet US 5.756.865 décrit l'obtention de méso-érythritol à partir d'acide L-tartrique, sucre oxydé de type acide aldarique coproduit par les industries de la vinification. Cette conversion se fait via des opérations d'hydrogénation, d'isomérisation et de séparation, les deux premières étant effectuées à 150°C et en présence de ruthénium, sous 80 bars de pression d'hydrogène. La sélectivité de la réaction d'hydrogénation n'apparait pas, là encore, satisfaisante.

Les demandes de brevet WO 97/49658 et WO 97/49659 divulguent la production de xylitol spécifiquement à partir d'un mélange initial contenant xylose et acide xylonique, à savoir une liqueur sulfitique issue de la production industrielle de cellulose.

Selon le cas, la composition de sucre oxydé (acide xylonique) soumise à hydrogénation a été préalablement débarrassée (WO 97/49658) ou non (WO 97/49658) du sucre non oxydé (xylose) qu'elle contenait initialement.

Même lorsque l'acide xylonique a été purifié par séparation/cristallisation, un traitement d'hydrogénation mené à relativement basse température, à savoir 110°C comme décrit dans l'exemple 15 de la demande WO 97/49658, ne permet pas d'atteindre une très grande sélectivité de conversion en xylitol, compte-tenu notamment de la formation concomitante d'arabitol.

La demande EP 820.979 décrit un procédé de préparation d'arabitol à partir de glucose imposant des étapes intermédiaires d'obtention, par décarboxylation oxydative, puis de cristallisation et de protonation d'un sel alcalin d'acide arabinonique.

L'étape obligatoire de cristallisation a pour finalité de débarrasser l'arabinonate de toute trace d'espèces, minérales ou organiques, aptes à empoisonner ensuite le catalyseur d'hydrogénation. Ces espèces consistent notamment en sels d'autres acides organiques (formiates, glycolates,...), générés lors de l'étape préalable d'oxydation catalytique, et en promoteurs rédox mis en oeuvre lors de cette étape.

Les auteurs insistent tout particulièrement sur l'impérieuse nécessité d'enlever totalement de l'acide arabinonique, outre les formiates, toute trace résiduelle du sel de sodium de l'acide 2-anthraquinone monosulfonique ou "AMS" qui aurait pu être utilisé auparavant comme promoteur rédox, conformément au brevet FR 2.722.200 accordé à la Demanderesse.

Les essais décrits dans le brevet EP 820.979 semblent cependant montrer qu'il serait particulièrement difficile, voire quasi-impossible, de débarrasser entièrement l'acide arabinonique des traces d'AMS dès lors que ce promoteur aurait été utilisé lors de l'étape préalable de décarboxylation catalytique du glucose. En suite de quoi, l'acide arabinonique soumis à hydrogénation doit, selon les auteurs, être non seulement exempt de formiates mais également avoir été obtenu en l'absence d'AMS. Ceci permettrait, selon les enseignements de l'exemple 3 dudit brevet, d'hydrogéner avantageusement le sucre oxydé à 135°C plutôt qu'à 150°C, température préconisée pour la conversion d'un sucre oxydé non totalement purifié. Il ne ressort pas clairement dudit exemple 3 quel(s) traitement(s) de purification éventuel(s) a pu exactement subir, avant hydrogénation, tout substrat originellement obtenu en présence d'AMS et référencé « +AMS » au niveau du tableau 3 de cet exemple.

Par là même, il ne peut être déduit clairement de cet exemple, le taux effectif d'AMS contenu dans un tel produit au moment de son hydrogénation.

En outre, ledit exemple 3 (y compris le tableau 3) ne permet pas de distinguer, parmi les pentitols obtenus à l'issue de l'étape d'hydrogénation, la part spécifiquement représentée par le produit souhaité, en l'occurrence l'arabitol.

En tout état de cause, dans l'hypothèse où l'arabitol constituerait la totalité desdits pentitols, la meilleure sélectivité résulterait du dernier essai décrit dans le tableau 3 pour lequel sont annoncés :
- un taux d'arabinonate résiduel de 2%, soit un taux de conversion de 98%,
- un taux global de pentitols de 92%,
- un taux global de produits de « cracking » de 6%.

En suite de quoi, selon les exemples du brevet EP 820 979, la sélectivité maximale théorique en arabitol serait de (92 : 92+6) X 100, soit de 93,9% environ.

Selon ce même document, la mise en oeuvre d'acide phosphorique ou borique lors de l'opération d'hydrogénation serait par contre bénéfique à cette opération sans que n'apparaisse clairement le taux de tels acides devant être introduit dans le milieu réactionnel et l'effet de ces acides sur la sélectivité de conversion de l'acide arabinonique en le pentitol souhaité, i.e l'arabitol, notamment en regard des autres pentitols produits lors de l'hydrogénation.

Il résulte de ce qui précéde qu'il existait un besoin de trouver un moyen de conversion des sucres oxydés en sucres hydrogénés par hydrogénation catalytique qui, simultanément :
- présente une très bonne sélectivité,
- soit applicable à des compositions de sucres oxydés d'origines et de natures très diverses,
- soit applicable à des sucres oxydés purifiés mais également à des substrats non totalement purifiés et notamment non totalement exempts de traces d'espèces pourtant considérées comme des poisons des catalyseurs d'hydrogénation, et
- soit applicable à des conditions très variées d'hydrogénation, notamment en termes de températures, pressions et catalyseurs d'hydrogénation.

Et le mérite de la Demanderesse est d'avoir trouvé, après de nombreuses recherches, qu'un tel moyen pouvait consister à hydrogéner catalytiquement les sucres oxydés en leurs sucres hydrogénés correspondants en présence de composés particuliers permettant notamment d'améliorer la sélectivité de la réaction.

De manière plus précise, la présente invention a pour objet un procédé de conversion de sucres oxydés en sucres hydrogénés comprenant une étape au cours de laquelle on soumet une composition contenant au moins un sucre oxydé à un traitement d'hydrogénation catalytique, caractérisé en ce que l'on augmente, de préférence d'au moins 0,5%, la sélectivité dudit traitement en effectuant celui-ci en présence d'un promoteur de sélectivité constitué par une base de Lewis.

La notion de "sucre" n'est aucunement limitative et comprend l'ensemble des monosaccharides, disaccharides, trisaccharides, oligosaccharides et polysaccharides, linéaires, cycliques ou branchés, ainsi que les mélanges de ces produits tels que les hydrolysats d'amidon.

Par "sucre oxydé" au sens de la présente invention, on entend l'ensemble des produits résultant de l'oxydation, au moins partielle, de quelque manière que ce soit, en une ou plusieurs étapes, en un ou plusieurs endroits, des sucres précités. Il s'agit avantageusement des produits d'oxydation des monosaccharides, en particulier des tétroses, pentoses et hexoses, et des disaccharides, et plus particulièrement des acides aldoniques, céto-aldoniques, aldariques et uroniques correspondant à l'érythrose, au thréose, au sorbose, au xylose, à l'arabinose, au ribose, au ribulose, au xylulose, au glucose, au galactose, au fructose, au mannose, au maltose ou au lactose, ainsi que les esters et les lactones pouvant correspondre auxdits acides.

Le "sucre oxydé" peut notamment être choisi dans le groupe comprenant les acides érythronique, tartrique, thréonique, ribonique, xylonique, xylarique, xyluronique, glucuronique, arabinonique, 4 cétoarabinonique, arabinarique, gluconique, 2 céto ou 5 cétogluconique, 2,5 dicétogluconique, glucarique, 2 cétogulonique, galactonique, galactarique, maltobionique ou lactobionique, les lactones et esters pouvant correspondre auxdits acides et les mélanges quelconques d'au moins deux quelconques de ces produits.

La composition de sucre(s) oxydé(s) soumise à hydrogénation peut avantageusement contenir de l'acide xylonique et/ou de l'acide arabinonique et/ou l'une au moins de leurs lactones.

Il peut notamment s'agir d'acide xylonique ou d'acide arabinonique obtenu par décarboxylation oxydative en présence d'AMS selon les enseignements du brevet FR 2.722.200 précité. Cette composition peut, en outre, contenir au moins un sucre non oxydé, ledit sucre non oxydé pouvant ou non être hydrogéné et pouvant ou non correspondre au sucre oxydé contenu dans ladite composition.

A titre d'exemples la composition soumise à hydrogénation peut consister en un mélange contenant, en proportions variées, a) acide xylonique (et/ou l'une de ses lactones) et xylose comme décrit dans la demande WO 97/49658 précitée ou b) acide arabinonique (et/ou l'une de ses lactones) et arabinose ou arabitol ou c) acide 4 cétoarabinonique (et/ou l'une de ses lactones) et xylose ou xylitol.

La matière sèche ("MS") de la composition de sucre(s) oxydé(s) soumise à hydrogénation n'est pas un paramètre limitatif du procédé objet de l'invention. De préférence, cette MS est d'au moins 10% et, notamment, comprise entre 15 et 50%.

Conformément au procédé selon la présente invention, le traitement d'hydrogénation catalytique est effectué en présence d'un promoteur de sélectivité constitué par une base de Lewis.

Par "promoteur de sélectivité", on entend ici tout composé permettant, pour le moins, d'augmenter la sélectivité du traitement d'hydrogénation, c'est-à-dire de favoriser la formation spécifique du sucre hydrogéné souhaité, par exemple la formation de xylitol à partir d'acide xylonique (et/ou de l'une de ses lactones), ou d'arabitol à partir d'acide arabinonique (et/ou de l'une de ses lactones).

Cette sélectivité est, de préférence, augmentée d'au moins 0,5% en regard de la valeur de sélectivité obtenue en absence de base de Lewis.

Elle peut notamment être augmentée de 0,5 à 6% environ et en particulier de 0,8 à 5% environ, comme il résulte des exemples décrits ci-après.

La sélectivité peut avantageusement être évaluée au moment où le sucre oxydé servant de substrat a été converti à raison de 98% environ de son poids et ce, indépendamment, en particulier, du nombre et de la nature des produits de conversion obtenus.

Une telle sélectivité, mesurée donc à un haut taux de conversion, est désignée ci-après par « sélectivité HTC ».

Et il est remarquable de souligner, comme il sera exemplifié ci-après, qu'en vue de la préparation d'un sucre hydrogéné de type pentitol comme l'arabitol, la présence d'une base de Lewis lors de l'hydrogénation du pentose oxydé de départ (acide arabinonique et/ou ses lactones, par exemple), permet d'obtenir :
- des valeurs de sélectivité HTC supérieures à 94%, en particulier comprises entre 94,5 et 99%, et donc supérieures à la valeur maximale théorique précitée de 93,9% pouvant être déduite du tableau 3 du brevet EP 820 979, et
- des augmentations de sélectivité HTC supérieures à 1%, en particulier comprises entre 1,2 et 5%.

Par "base de Lewis", on entend classiquement tout composé ayant la capacité de pouvoir céder un ou plusieurs doublets électroniques.

D'une manière générale, le promoteur de sélectivité est avantageusement choisi parmi les quinones, en particulier l'acide anthraquinone-2 sulfonique et ses sels, les amines, grasses ou non grasses, telles que l'aniline et la triéthylamine, les phosphines telles que la tri-n butyl phosphine et la triphényl phosphine, et les mélanges quelconques d'au moins deux quelconques de ces produits.

De façon préférentielle, ce promoteur est constitué, en tout ou partie, d'acide anthraquinone-2 sulfonique et/ou d'au moins un sel dudit acide, et notamment de son sel sodique ou "AMS".

Il est remarquable de noter que c'est en prenant totalement le contre-pied des enseignements du brevet EP 820.979 susmentionné que la Société Demanderesse a constaté, à son grand étonnement, que non seulement un produit comme l'AMS pouvait ne pas être un "poison" de catalyseur d'hydrogénation, mais encore que sa présence lors de l'opération d'hydrogénation permettait d'augmenter significativement la sélectivité de ladite opération.

Sans vouloir être lié par une quelconque théorie, on peut avancer l'hypothèse selon laquelle une base de Lewis serait apte à modifier l'environnement électronique et/ou stérique des particules métalliques du catalyseur d'hydrogénation et ainsi susceptible de diminuer significativement, voire d'inhiber, les réactions secondaires, en particulier les réactions d'hydrogénolyse, génératrices de produits indésirables jouant négativement sur la sélectivité de la réaction principale d'hydrogénation.

Il convient également de signaler que les modalités d'introduction du promoteur de sélectivité dans le milieu réactionnel peuvent être très variées. La base de Lewis peut être, en tout ou partie, déjà présente dans la composition de sucre(s) oxydé(s) devant être hydrogénée et/ou être amenée, en tout ou partie, indépendamment de ladite composition, que ce soit avant, après et/ou simultanément à celle-ci.

En suite de quoi, l'un des intérêts indéniables du procédé conforme à l'invention est d'être applicable à des sucres oxydés non obligatoirement parfaitement purifiés et notamment à des produits obtenus en présence d'AMS conformément au brevet FR 2.722.200 et qui n'auraient pas été totalement débarrassés des traces résiduelles d'AMS, lesquelles seraient donc présentes lors de l'hydrogénation.

Selon une variante préférentielle du procédé selon l'invention, le traitement d'hydrogénation catalytique est effectué en présence de 100 à 4000 ppm, de préférence de 100 à 3000 ppm, d'un tel promoteur de sélectivité, ces concentrations étant exprimées en poids sec de promoteur par rapport au poids sec du milieu réactionnel.

De façon particulièrement avantageuse, ce traitement se fait en présence de 350 à 2500 ppm, et notamment de 400 à 1500 ppm, d'un tel promoteur de sélectivité.

Selon une autre variante du procédé, le traitement d'hydrogénation catalytique est effectué à une température inférieure à 130°C, notamment comprise entre 75 et 125°C environ, et à une pression d'hydrogène comprise entre 60 et 150 bars environ.

De telles pressions se distinguent des pressions relativement basses (40 bars) utilisées dans les exemples du brevet EP 820.979 précité.

D'autre part, de manière avantageuse, le catalyseur d'hydrogénation est à base, au moins, d'un métal choisi parmi le ruthénium, le rhénium, l'iridium et les mélanges quelconques d'au moins deux quelconques de ces métaux et tout particulièrement à base d'un métal constitué, en tout ou partie, de ruthénium.

La Société Demanderesse a par ailleurs observé, comme il sera exemplifié par la suite, que le catalyseur d'hydrogénation peut avantageusement avoir été préparé par une technique quelconque d'échange ionique plutôt que par imprégnation.

L'utilisation d'un catalyseur ainsi préparé, en vue de l'hydrogénation catalytique de sucre(s) oxydé(s), est nouvelle à la connaissance de la Demanderesse et fait partie de la présente invention. Il en est de même de l'utilisation, aux mêmes fins, d'un catalyseur préparé par une technique quelconque d'apport d'un promoteur de sélectivité tel que décrit. Ainsi le promoteur peut être apporté sur un catalyseur métallique par dépôt en surface ou imprégnation du support ou par tout autre moyen et ce, simultanément ou non à l'apport du métal sur ledit support. Ce dernier peut être constitué par exemple, de charbon actif, de tourbe, de zéolithe ou d'un polymère synthétique de type fibre de carbone.

En suite de quoi, on dispose désormais d'un nouveau moyen, particulièrement sélectif, de préparation de sucres hydrogénés tels que ceux susmentionnés, cette remarquable sélectivité étant liée à la présence de base(s) de Lewis lors du traitement d'hydrogénation mis en oeuvre pour cette préparation.

Ce moyen procède globalement du concept inventif ayant pour objet l'utilisation d'une base de Lewis en tant que promoteur de sélectivité d'une réaction d'hydrogénation catalytique d'un sucre oxydé.

Il peut notamment être utilisé pour la préparation d'un sucre hydrogéné compris dans le groupe comprenant l'érythritol, le thréitol, le ribitol, le xylitol, l'arabitol, le mannitol, le sorbitol, l'iditol, le maltitol, le lactitol et les mélanges quelconques d'au moins deux quelconques de ces produits.

A la connaissance de la Demanderesse et compte-tenu notamment des enseignements du brevet EP 820.979 susmentionné, personne n'avait jusqu'ici envisagé de conduire un procédé d'hydrogénation catalytique de sucre(s) oxydé(s) en présence de quantités élevées, i.e d'au moins 350 ppm environ, d'une base de Lewis comme l'AMS, produit par ailleurs connu pour ses effets néfastes sur les catalyseurs d'hydrogénation.

Or non seulement un tel procédé est nouveau mais encore il permet, comme il sera exemplifié ci-après, de produire des effets surprenants, en particulier en termes de sélectivité de réaction, effets qui, de manière remarquable, se perpétuent tout au long de la réaction d'hydrogénation.

Un tel procédé fait également partie de la présente invention, laquelle va être décrite de façon encore plus détaillée à l'aide des exemples qui suivent et qui ne sont aucunement limitatifs.

### EXEMPLE 1

Une composition de sucre oxydé consistant en une solution à 20% de matière sèche ("MS") d'acide arabinonique pur, est soumise à un traitement d'hydrogénation dans les conditions suivantes :
- température : 100°C,
- pression d'hydrogène : 100 bars,
- catalyseur : ruthénium sur charbon actif :
   . charge en métal : 5%,
   . technique de préparation : imprégnation,
   . surface BET : 900 m²/g,
   . taille des particules : 88 µm environ.
- quantité de catalyseur : 0,0375g/g sec de sucre oxydé.

On mesure, au cours du temps, les paramètres suivants :
1) le taux de conversion, i.e le pourcentage, en poids, de sucre oxydé (acide arabinonique) converti en le produit hydrogéné souhaité (arabitol) et en produits non souhaités, y compris en autres pentitols (dont xylitol, ribitol, déoxypentitols).
2) le taux de sélectivité, i.e le pourcentage, en poids, de sucre hydrogéné souhaité (arabitol) obtenu par rapport au sucre oxydé (acide arabinonique) converti.

Les résultats ci-après font apparaître le taux de sélectivité obtenu en fonction du taux de conversion de l'acide arabinonique, i.e en fonction du déroulement de l'étape d'hydrogénation.

| | | | | |
|---|---|---|---|---|
| Taux de conversion (%) | 20 | 60 | 80 | 98 |
| Taux de sélectivité (%) | 95,0 | 94,8 | 94,2 | 93,6 |

Un essai mené dans les mêmes conditions mais en doublant la quantité de catalyseur n'a pas eu globalement d'effet significatif sur le taux de sélectivité.

Dans un second essai, la même composition de sucre oxydé que celle décrite ci-dessus est hydrogénée dans les mêmes conditions que celles énumérées ci-avant, si ce n'est que l'on introduit dans le milieu réactionnel avant hydrogénation, respectivement, 500, 1000 ou 2000 ppm du sel de sodium de l'acide anthraquinone-2 sulfonique ou "AMS", ces proportions étant exprimées en poids sec d'AMS par rapport au poids sec du milieu réactionnel.

On obtient les résultats suivants de -taux de sélectivité (TS en %) en fonction du taux de conversion (TC en %) et du taux d'AMS introduit (en ppm).

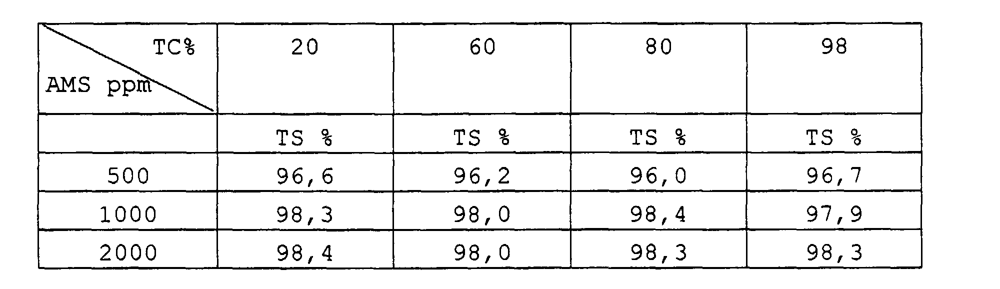

On observe que l'introduction d'une base de Lewis comme l'AMS permet, globalement, d'augmenter le taux de sélectivité de la conversion de l'acide arabinonique en arabitol.

Dans le cas présent, cette augmentation de sélectivité est d'au moins 1,5%, valeur ici obtenue en tenant compte des valeurs de sélectivité mesurées respectivement en absence d'AMS (94,8%) et en présence de 500 ppm d'AMS (96,2%), pour un taux de conversion de 60%.

Cette augmentation de sélectivité peut atteindre 5% environ, valeur ici obtenue en tenant compte des valeurs de sélectivité mesurées respectivement en absence d'AMS (93,6%) et en présence de 2000 ppm d'AMS (98,3%), pour un taux de conversion de 98% (sélectivités HTC).

Et il est remarquable de noter que, d'une manière générale, cette amélioration de sélectivité se perpétue tout au long du traitement d'hydrogénation, y compris dans les dernières phases de conversion du sucre oxydé (cf résultats obtenus à 80 et 98% de conversion).

Cette amélioration générale a par ailleurs été vérifiée par la Société Demanderesse à des températures d'hydrogénation allant jusqu'à 140°C au moins. Cependant, de façon absolue, il a été trouvé que la meilleure sélectivité, en présence d'une base de Lewis comme l'AMS était obtenue pour des températures d'hydrogénation inférieures à 130°C, se situant notamment entre 75 et 125°C environ.

### EXEMPLE 2

Dans cet exemple, on étudie les effets, sur le taux de sélectivité, de la présence de 1000 ppm, respectivement, de triéthylamine ou d'aniline, lors du traitement d'hydrogénation.

Ce traitement est effectué dans les conditions décrites pour l'EXEMPLE 1 si ce n'est que, dans le cas présent, on utilise un catalyseur (5% de ruthénium sur charbon actif, préparé par imprégnation) se distinguant de celui de l'EXEMPLE 1 par ses caractéristiques de surface BET (1100 m²/g) et de taille des particules (85 µm environ).

En tant qu'essai "témoin", on hydrogène la solution à 20% de MS d'acide arabinonique pur, en présence dudit catalyseur et en absence de toute base de Lewis.

On obtient les résultats ci-après de taux de sélectivité (TS en %) en fonction du taux de conversion (TC en %) et de la nature de la base de Lewis éventuellement introduite.

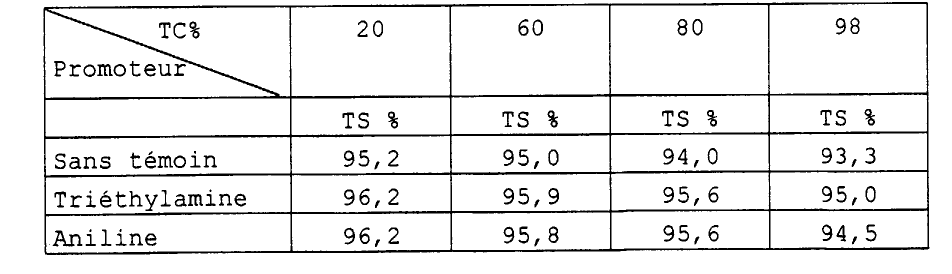

Cet exemple montre que des bases de Lewis comme les amines peuvent également être valablement utilisées comme promoteurs de sélectivité dans le cadre de l'invention.

### EXEMPLE 3

On prépare un catalyseur par technique d'échange ionique, en l'occurrence cationique, selon le schéma global ci-après. A 20,3 g de charbon actif de type "Norit SX Ultra" (1250 m²/g) on ajoute 200 ml d'ammoniaque 1M. On ajoute ensuite 1,55 g de sel de ruthénium, à savoir de [Ru (NH₃)6] Cl₃, et 50 ml d'ammoniaque. Après 24 heures d'agitation et bullage d'azote puis des étapes de filtration, lavage, séchage, réduction sous hydrogène (3h30 à 300°C) et enfin de passivation (3h avec 1% O₂/N₂), on obtient un catalyseur contenant 2,5% environ de ruthénium et présentant une taille de particules inférieure à 1 nm.

En tant que premier essai témoin, on hydrogène une solution à 20% de MS d'acide arabinonique pur, en présence du catalyseur tel que préparé et en absence de toute base de Lewis. Ceci, conformément au protocole décrit à l'EXEMPLE 1.

Dans ce cas, on obtient tout au long de la réaction, une sélectivité d'environ 97%, cette sélectivité baissant très légèrement (96,3%) en fin de réaction, i.e pour la valeur du taux de conversion de 98%. Ce premier essai témoin montre qu'en regard d'un catalyseur préparé par une technique d'imprégnation (cf EXEMPLES 1 et 2), un catalyseur préparé par une technique d'échange ionique est significativement plus performant en termes de sélectivité de réaction.

Dans le cadre du même exemple, on réalise un second essai témoin identique au premier si ce n'est que :
1) on met en oeuvre 2,5 fois plus de catalyseur préparé par technique d'échange ionique, à savoir 2,5 x 0,0375 = 0,094g de catalyseur/g sec de sucre oxydé, et
2) on abaisse la température d'hydrogénation à 80°C.

La sélectivité obtenue est d'environ 98% et ce, tout au long de la réaction avec, entre autres, une valeur de 98,1% pour un taux de conversion de 98%. L'augmentation de la sélectivité HTR est donc, dans le cas présent, de (98,1 - 96,3) : 96,3 X 100 soit d'environ 1,9%.

Le même essai réalisé à 80°C mais en présence de 0,0375g de catalyseur/g sucre oxydé et avec introduction de 1000 ppm d'AMS permet d'atteindre et de maintenir lors de la réaction une sélectivité d'environ 99% avec, entre autres, une valeur de 98,9% pour un taux de conversion de 98%.

Ces résultats montrent, outre l'intérêt général d'employer un catalyseur préparé par technique d'échange ionique dans le cadre de l'hydrogénation de sucre(s) oxydé(s), que le procédé conforme à l'invention permet, de par la mise en oeuvre de bases de Lewis comme l'AMS, d'atteindre et de maintenir des taux de sélectivité particulièrement élevés, y compris égaux ou supérieurs à 99%.

Le procédé selon l'invention permet donc notamment d'obtenir un sucre hydrogéné de manière tant qualitative que quantitative à partir d'un sucre oxydé et ce, sans faire obligatoirement appel ni à un substrat (sucre oxydé) totalement purifié, ledit substrat pouvant contenir des traces d'AMS, ni à une étape subséquente visant à séparer ledit sucre hydrogéné des autres composés (en particulier isomères et produits de dégradation) classiquement générés lors de la réaction d'hydrogénation.

## Revendications

1. Procédé de conversion de sucres oxydés en sucres hydrogénés comprenant une étape au cours de laquelle on soumet une composition contenant au moins un sucre oxydé à un traitement d'hydrogénation catalytique, caractérisé en ce que l'on augmente, de préférence d'au moins 0,5%, la sélectivité dudit traitement en effectuant celui-ci en présence d'un promoteur de sélectivité constitué par une base de Lewis.

2. Procédé selon la revendication 1 caractérisé en ce que :
a) le promoteur de sélectivité est choisi dans le groupe comprenant les quinones, en particulier l'acide anthraquinone-2 sulfonique et ses sels, les amines, grasses ou non grasses, telles que la triéthylamine et l'aniline, les phosphines telles que la tri-n butyl phosphine et la triphényl phosphine et les mélanges quelconques d'au moins deux quelconques de ces produits, et
b) le catalyseur utilisé pour le traitement d'hydrogénation est à base, au moins, d'un métal choisi parmi le ruthénium, le rhénium, l'iridium et les mélanges quelconques d'au moins deux quelconques de ces métaux.

3. Procédé selon l'une ou l'autre des revendications 1 et 2 caractérisé en ce que :
a) le promoteur est constitué, en tout ou partie, d'acide anthraquinone sulfonique et/ou d'au moins un sel dudit acide, notamment du sel sodique dudit acide, et
b) le catalyseur est à base d'un métal constitué, en tout ou partie, de ruthénium.

4. Procédé selon l'une quelconque des revendications 1 à 3 caractérisé en ce que le catalyseur utilisé pour le traitement d'hydrogénation catalytique a été préparé par une technique quelconque d'échange ionique et/ou par une technique quelconque d'apport d'un promoteur de sélectivité constitué par une base de Lewis.

5. Procédé selon l'une quelconque des revendications 1 à 4 caractérisé en ce que le traitement d'hydrogénation catalytique est effectué à une température inférieure à 130°C, notamment comprise entre 75 et 125°C environ, et à une pression d'hydrogène comprise entre 60 et 150 bars environ.

6. Procédé selon l'une quelconque des revendications 1 à 5 caractérisé en ce que le traitement d'hydrogénation catalytique est effectué en présence de 100 à 4000 ppm, de préférence de 100 à 3000 ppm, d'un promoteur de sélectivité, ces concentrations étant exprimées en poids sec de promoteur par rapport au poids sec du milieu réactionnel.

7. Procédé selon la revendication 6 caractérisé en ce que le traitement d'hydrogénation catalytique est effectué en présence de 350 à 2500 ppm, notamment de 400 à 1500 ppm, d'un promoteur de sélectivité.

8. Procédé selon l'une quelconque des revendications 1 à 7 caractérisé en ce que la composition soumise au traitement d'hydrogénation catalytique contient au moins un sucre oxydé choisi dans le groupe comprenant les produits d'oxydation des monosaccharides, en particulier des tétroses, pentoses et hexoses, et des disaccharides, et plus particulièrement choisi parmi les acides aldoniques, céto-aldoniques, aldariques et uroniques correspondant à l'érythrose, au thréose, au sorbose, au xylose, à l'arabinose, au ribose, au ribulose, au xylulose, au glucose, au galactose, au fructose, au mannose, au maltose ou au lactose, ainsi que les esters et les lactones pouvant correspondre auxdits acides.

9. Utilisation du procédé selon l'une des revendications 1 à 8 pour la préparation d'un sucre hydrogéné compris dans le groupe comprenant l'érythritol, le thréitol, le ribitol, le xylitol, l'arabitol, le mannitol, le sorbitol, l'iditol, le maltitol, le lactitol et les mélanges quelconques d'au moins deux quelconques de ces produits.

10. Utilisation, en vue de l'hydrogénation catalytique de sucre(s) oxydé(s), d'un catalyseur à base, au moins, de ruthénium, de rhénium et/ou d'iridium, ledit catalyseur ayant été préparé par une technique quelconqque d'échange ionique et/ou par une technique quelconque d'apport d'un promoteur de sélectivité constitué par une base de Lewis.

11. Procédé d'hydrogénation catalytique de sucre(s) oxydé(s) caractérisé en ce qu'il est effectué en présence d'au moins 350 ppm environ d'une base de Lewis, de préférence choisie dans le groupe comprenant les quinones, en particulier l'acide anthraquinone-2 sulfonique et ses sels, les amines, grasses ou non grasses, telles que la triéthylamine et l'aniline, les phosphines telles que la tri-n butyl phosphine et la triphényl phosphine et les mélanges quelconques d'au moins deux quelconques de ces produits, cette concentration étant exprimée en poids sec de base de Lewis par rapport au poids sec du milieu réactionnel.

12. Utilisation d'une base de Lewis en tant que promoteur de sélectivité d'une réaction d'hydrogénation catalytique d'un sucre oxydé.
